# EUROPEAN PATENT SPECIFICATION

(11) **EP 3 328 343 B1**
(45) Date of publication and mention of the grant of the patent: **17.07.2019**
(21) Application number: 16744387.8
(22) Date of filing: 27.07.2016
(51) Int. Cl.: A61K 8/00

(54) **ANTI-HAIR LOSS LOTION**
LOTION ZUR BEHANDLUNG VON HAARAUSFALL
LOTION POUR LE TRAITEMENT DE LA CHUTE DES CHEVEUX

(30) Priority: 31.07.2015 EP 15382409
(43) Date of publication of application: 06.06.2018
(73) Proprietor: Isdin, S. A., 08019 Barcelona (ES)
(72) Inventor: TRULLÀS CABANAS, Carles, 08019 Barcelona (ES); FOYACA FERRER, Monica, 08019 Barcelona (ES)
(74) Representative: ZBM Patents - Zea, Barlocci & Markvardsen
(86) International application number: PCT/EP2016/067869
(87) International publication number: WO 2017/021247

(56) References cited:
- WO-A1-2007/046577
- DE-A1- 10 250 646
- US-B1- 7 514 474

## Description

The present invention relates to a topical composition. Particularly, the invention relates to an anti-hair loss topical composition comprising diaminopyrimidine oxide, oleanolic acid, biotinoyl tripeptide-1, apigenin, biotin, adenosine or an adenosine derivative, and Gingko biloba and to the composition for use in reducing or preventing hair loss.

### BACKGROUND ART

Hair loss or alopecia is a common and embarrassing problem for many people. Individuals suffering hair loss often experience emotional distress due to their change in appearance.

One of the most frequent types of alopecia is androgenetic alopecia which is caused by male hormones and hereditary background, and which act on hair follicles causing their progressive miniaturisation until atrophy occurs. Nevertheless, besides hormones, there are many other factors that can promote the complex phenomenon of hair loss, such as ageing, physiological stress, and deficiency in nutrition of the hair, among many others.

For many years, considerable research has been conducted to develop compositions for reducing hair loss or even stimulating its growth. For this purpose, a large number of active substances, usually acting by different mechanisms, are known. Among them vasodilators such as potassium channel agonists including minoxidil and minoxidil derivatives such as diaminopyrimidine oxide; and 5-alpha reductase inhibitors, such as finasteride has been used. Many other substances such as adenosine, biotin, apigenin, oleanoic acid, biotinoyl tripeptide, and extracts of hop, rosemary, Swertia, horsetail, safflower, stinging nettle, Gingko biloba, Aloe vera, Serenoa serrulata, and gingseng have also been used in cosmetic or pharmaceutical formulations for reducing hair loss or stimulating its growth. Nevertheless, the use of additives necessary to obtain stable formulations, lead that formulations that are currently in the market leave a greasy or dirty appearance on the hair.

Another main problem of the existing treatments is that their interruption is followed by gradual hair loss returning to the pretreatment situation. As a consequence, it is not possible to stop the treatment.

Thus, despite the possible ways known in the prior to obtain effective compositions for reducing hair loss or even stimulating its growth, the provision of stable compositions having the mentioned activity with a longer remaining effect and with improved sensorial attributes goes on being an active field of research.

### SUMMARY OF THE INVENTION

The present inventors, in an attempt for developing an improved anti-hair loss topical formulation, have found a combination of components that provides a stable composition providing a reduction of hair loss during the treatment and having a long lasting effect after the discontinuation of the treatment.

Diaminopyrimidine oxide (trade name Aminexil) is a chemical compound similar to minoxidil, also used for therapy of alopecia.

Adenosine and adenosine derivatives, particularly adenosine 5'-phosphate derivatives, have also been disclosed for reducing hair loss. As used herein, an "adenosine 5'-phosphate derivative" refers to an adenosine compound in which the 5' carbon of the nucleoside sugar moiety is bound to at least one phosphate group, and pharmaceutically acceptable salts thereof. Phosphate groups can be mono-, di-, or tri-phosphates or derivatives thereof, such as 1-, 2-, and/or 3-thiotriphosphates. The adenosine 5'-phosphates used herein are typically adenosine 5'-monophosphate ("AMP"), adenosine 5'-diphosphate ("ADP"), adenosine 5'-triphosphate ("ATP") or pharmaceutically acceptable salts thereof. Other adenosine derivatives include, without being limited to, adenosine cyclic phosphate, methylthioadenosine, 3'-phosphoadenosine-5'-phosphosulfate, coenzyme A, and pharmaceutically acceptable salts thereof.

Biotinoyl tripeptide-1 is a Matrikine acting on the anchoring of the hair thanks to adhesion proteins.

Oleanolic acid inhibits the transformation of testosterone into dihydrotestosterone via 5α-reductase and thus may be advantageously used in the treatment of hair loss.

Apigenin is a citrus extract flavonoid with a vasodilatory effect. Its use on the scalp allows for a greater flow of blood to the hair follicles, promoting healthy hair growth.

Biotin is a water-soluble B-vitamin (vitamin B7). The hair growth promoting effects of biotin has been known for a long time.

Extracts of leaves and seeds of Gingko biloba have been used for many years. Particularly, extracts obtained from Ginkgo leaves have been disclosed as useful in stimulating hair growth.

Although it is known that the abovementioned active ingredient have some effect either on the reduction of hair loss or on stimulating its growth, their combined use has not been disclosed. It has surprisingly been found that the addition of Gingko biloba to a composition comprising diaminopyrimidine oxide, oleanolic acid, biotinoyl tripeptide-1, apigenin, biotin, and adenosine or an adenosine derivative has an unexpected effect on the prevention or reduction of hair loss and on stimulating its growth which is not observed by the topical application of the same composition without Gingko biloba or even of other anti-hair loss compositions currently available in the market. This effect is higher than the sum of effects of the combination of diaminopyrimidine oxide, oleanolic acid, biotinoyl tripeptide-1, apigenin, biotin, and adenosine or an adenosine derivative, and of Gingko biloba, separately. Thus, the addition of Gingko biloba into a combination of diaminopyrimidine oxide, oleanolic acid, biotinoyl tripeptide-1, apigenin, biotin, and adenosine or an adenosine derivative provides a synergic effect on the reduction of hair loss or on stimulating its growth.

Also advantageously, by the combined effect of the mentioned active agents, a long-lasting effect is observed after the discontinuation of the treatment. Particularly, the effect of the composition on the reduction of hair loss is maintained during several months in spite of the interruption of the treatment when the daily application of the product is hindered by any reason.

Thus, in a first aspect the present invention relates to a combination comprising diaminopyrimidine oxide, oleanolic acid, biotinoyl tripeptide-1, apigenin, biotin, adenosine or an adenosine derivative, and Gingko biloba.

In a second aspect the present invention relates to a topical composition comprising a therapeutically effective amount of a combination comprising diaminopyrimidine oxide, oleanolic acid, biotinoyl tripeptide-1, apigenin, biotin, adenosine or an adenosine derivative, and Gingko biloba, together with appropriate topical cosmetically or pharmaceutically acceptable excipients or carriers.

The composition of the invention can be used topically on the scalp to reduce or prevent the loss of hair and/or stimulate its growth. Accordingly, another aspect the invention relates to a pharmaceutical composition as defined herein above or below for use in reducing or preventing the loss of hair and/or stimulating its growth, particularly in a man. This aspect can also be formulated as the use of the composition as defined above for the manufacture of a medicament for reducing or preventing the loss of hair and/or for stimulating its growth, particularly in a man. It also relates to a method for reducing or preventing the loss of hair and/or for stimulating its growth comprising administering a pharmaceutically effective amount of the composition of the invention to a subject, particularly a man, in need of the treatment.

### DETAILED DESCRIPTION OF THE INVENTION

All terms as used herein in this application, unless otherwise stated, shall be understood in their ordinary meaning as known in the art. Other more specific definitions terms as used in the present application are as set forth below and are intended to apply uniformly throughout the specification and claims unless an otherwise expressly set out definition provides a broader definition.

The term "lotion", as used herein, the term "lotion" refers to any liquid preparation, particularly to a hydroalcoholic liquid formulation.

The term "cosmetically acceptable" as used herein, means that the compositions or components thereof so-described are suitable for use in contact with human skin without undue toxicity, incompatibility, instability, allergic response, or the like.

The expression "pharmaceutically acceptable" as used herein, means that the components so-described are compatible with the other ingredients of the pharmaceutical composition, and are suitable for use in contact with human skin without undue toxicity, incompatibility, instability, allergic response, or the like.

The expression "therapeutically effective amount" as used herein, refers to the amount of a compound that, when administered, is sufficient to prevent development of, or alleviate to some extent, one or more of the symptoms of the disorder or condition being treated. The particular dose of compound administered according to this invention will of course be determined by the particular circumstances surrounding the case, including the particular condition being treated, the duration of the treatment, the nature of any concurrent treatment, the specific combination of active ingredient used, and any other factor known to the expert.

The term "percentage by weight" or "wt. %" refers to the percentage by weight of the ingredient per weight of the overall composition.

As mentioned above, the combination of the invention comprises diaminopyrimidine oxide, oleanolic acid, biotinoyl tripeptide-1, apigenin, biotin, adenosine or an adenosine derivative, and Gingko biloba in the weight ratios mentioned above. It is particularly suitable for the preparation of formulations for topical application on the hair.

Weight ratios of the different components in the composition correspond to the weight ratios of the same components in the combination.

In a particular embodiment of the composition of the invention, optionally in combination with one or more features of the particular embodiments defined above, the weight ratio of the combination diaminopyrimidine oxide, oleanolic acid, biotinoyl tripeptide-1, apigenin, biotin and adenosine or an adenosine derivative to Ginkgo biloba is from 60:1 to 15:1, particularly from 50:1 to 20:1.

In another particular embodiment, the composition of the invention is a lotion wherein the carrier is a mixture of water and ethanol, the water being present in an amount from 25 wt. % to 70 wt. % of the total composition, and the ethanol being present in an amount from 75 wt. % to 30 wt. % of the total composition.

The use of the composition of the invention in the form of lotion comprising ethanol/water is specially advantageous since the specific combination of diaminopyrimidine oxide, oleanolic acid, biotinoyl tripeptide-1, apigenin, biotin, adenosine or an adenosine derivative, Gingko biloba, water and ethanol allows obtaining a stable anti-hair loss topical lotion providing a non-oily, non-sticky feeling when applied on the scalp. The liquid topical composition has a fluid, watery texture providing an intense non-greasy, non-sticky, fresh feeling. Additionally, the composition of the invention when is in the form of a lotion, slides soft and pleasant by massaging and is absorbed by the skin very quickly after its application, leaving a hair with a dry, clean, loose and flowing appearance. As a consequence, the product can be applied as many times as necessary, without the limitation of aesthetic concerns.

In another particular embodiment of the composition of the invention, optionally in combination with one or more features of the particular embodiments defined above, the amount of diaminopyrimidine oxide is from 0.2 wt. % to 2 wt. %, more particularly from 0.4 wt. % to 1.5 wt. %, of the total composition.

In another particular embodiment of the composition of the invention, optionally in combination with one or more features of the particular embodiments defined above, the amount of oleanolic acid is from 0.00001 wt. % to 0.1 wt. %, more particularly from 0.0001 wt. % to 0.01 wt. %, of the total composition.

In another particular embodiment of the composition of the invention, optionally in combination with one or more features of the particular embodiments defined above, the amount of biotinoyl tripeptide-1 is from 0.00001 wt. % to 0.1 wt. %, more particularly from 0.0001 wt. % to 0.01 wt, of the total composition.

In another particular embodiment of the composition of the invention, optionally in combination with one or more features of the particular embodiments defined above, the amount of apigenin is from 0.0001 wt. % to 0.01 wt. %, more particularly from 0.0005 wt. % to 0.01 wt. %, of the total composition.

In another particular embodiment, optionally in combination with one or more features of the particular embodiments defined above, the amount of biotin is from 0.001 wt. % to 0.1 wt. %, more particularly from 0.02 wt. % to 0.05 wt. %, more particularly from 0.001 wt. % to 0.05 wt. %, and even more particularly from 0.01 wt. % to 0.05 wt. %, of the total composition.

In another particular embodiment of the composition of the invention, optionally in combination with one or more features of the particular embodiments defined above, the amount of adenosine or adenosine derivative is from 0.1 wt. % to 1 wt. %, more particularly from 0.20 wt. % to 1 wt. %, of the total composition. In a particular embodiment, the composition of the invention comprises adenosine. In another particular embodiment, the composition of the invention comprises and adenosine 5'-phosphate derivative or a pharmaceutically acceptable salt thereof, more particularly adenosine 5'-triphosphate or a pharmaceutically acceptable salt thereof.

In another particular embodiment of the composition of the invention, optionally in combination with one or more features of the particular embodiments defined above, the amount of Gingko biloba is from 0.001 wt. % to 0.1 wt. %, more particularly from 0.01 wt. % to 0.08 wt. %, and even more particularly from 0.01 wt. % to 0.1 wt. %, of the total composition.

In another particular embodiment of the composition of the invention, optionally in combination with one or more features of the particular embodiments defined above, the amount of diaminopyrimidine oxide is from 0.2 wt. % to 2 wt. % of the total composition, the amount of oleanolic acid is from 0.00001 wt. % to 0.1 wt. % of the total composition, the amount of biotinoyl tripeptide-1 is from 0.00001 wt. % to 0.1 wt. % of the total composition, the amount of apigenin is from 0.0001 wt. % to 0.01 wt. % of the total composition, the amount of biotin is from 0.02 wt. % to 0.05 wt. % of the total composition, the amount of adenosine or adenosine derivative is from 0.1 wt. % to 1 wt. % of the total composition, and the amount of Gingko biloba is from 0.001 wt. % to 0.1 wt. % of the total composition.

In another particular embodiment of the composition of the invention, optionally in combination with one or more features of the particular embodiments defined above, the amount of diaminopyrimidine oxide is from 0.4 wt. % to 1.5 wt. % of the total composition, the amount of oleanolic acid is from 0.0001 wt. % to 0.01 wt. % of the total composition, the amount of biotinoyl tripeptide-1 is from 0.0001 wt. % to 0.01 wt. % of the total composition, the amount of apigenin is from 0.0005 wt. % to 0.01 wt. % of the total composition, the amount of biotin is from 0.001 wt. % to 0.05 wt. % of the total composition, the amount of adenosine or adenosine derivative is from 0.20 wt. % to 1 wt. % of the total composition, and the amount of Gingko biloba is from 0.01 wt. % to 0.1 wt. % of the total composition.

As mentioned above, in the composition of the invention weight ratios of the different components are expressed with respect to the total amount of components of the composition. In the combination, the weight ratios of the components can also be expressed with respect to the total amount of components of the combination. Thus, the weight ratios of the different components with respect to the total amount of components of the composition correspond to the weight ratios of the same components in the combination with respect to the total amount of components of the combination.

As a way of example, weight percentages of the active ingredients for the composition mentioned above would correspond to the following weight percentages of the components in the combination of the invention, where excipients or carriers are not included: 64.44 wt. % to 55.97 wt. % of diaminopyrimidine oxide, 0.016 wt. % to 0.37 wt. % of oleanolic acid, 0.016 wt. % to 0.37 wt. % of biotinoyl tripeptide-1, 0.08 wt. % to 0.37 wt. % of apigenin, 1.61 wt. % to 1.87 wt. % of biotin, 32.22 wt. % to 37.31 wt. % of adenosine or adenosine derivative, and 1.61 wt. % to 3.73 wt. % of Gingko biloba, the total amount of components being 100%. Similarly, the weigh percentages of the components in the combinations used in the rest of specific compositions disclosed herein can be also calculated.

The compositions of the present invention can comprise or consist of, the components described herein. Accordingly, in a particular embodiment, the topical composition consists of diaminopyrimidine oxide, oleanolic acid, biotinoyl tripeptide-1, apigenin, biotin, adenosine or an adenosine derivative, and Gingko biloba, together with appropriate topical cosmetically or pharmaceutically acceptable excipients or carriers.

As mentioned above, the composition of the invention can comprise one or more appropriate topical cosmetically or pharmaceutically acceptable excipient such as antioxidants, preservatives, humectants, pH-regulating agents, chelating agents, among others. The appropriate cosmetically or pharmaceutically excipients and/or carriers and their amounts can readily be determined by those skilled in the art.

Examples of antioxidants include, without being limited to, tocopheryl acetate, tocopherol, butylated hydroxytoluene (BHT), ascorbyl palmitate, and vitamin C.

Examples of preservatives include, without being limited to, phenoxyethanol, methylparaben, propylparaben, sodium benzoate.

Examples of humectants include, without being limited to, PEG-6, PEG-8, PEG-32, glycerine, propylene glycol, butylene glycol, sodium hyaluronate, and hyaluronic acid.

Examples of pH-regulating agents include, without being limited to, sodium hydroxide, potassium hydroxide, triethanolamine, citric acid, and lactic acid.

Examples of chelating agents include, without being limited to, EDTA.

Additionally, the composition of the present invention may contain other ingredients, such as fragrances, colorants, and other components known in the state of the art for use in topical formulations.

The composition of the invention as defined above can be conceived as a base composition suitable for pharmaceutical compounding of ingredients commonly used in pharmaceutical compositions for use in reducing loss of hair. Accordingly, the composition of the present invention can contain additional active ingredients to those already mentioned. In a particular embodiment, optionally in combination with one or more features of the particular embodiments defined above, the composition of the invention further comprises finasteride, minoxidil, clobetasol, triamcinolone, clindamycin, ketoconazole, salicylic acid, or a combination thereof. Advantageously, any one of the mentioned additional active ingredients or a combination thereof can be incorporate in the composition of the invention without affecting to its stability, i.e. it is obtained a stable composition while maintaining the improved sensorial attributes of the base composition.

Finasteride is a drug known for the treatment of male androgenetic alopecia The drug, commercialized for oral administration, reduces loss of hair by inhibiting 5-α reductase enzyme activity, which converts testosterone to its active form, namely di-hydrotestosterone (DHT), which is the main cause of male pattern hair loss. The physical chemical properties of Finasteride make difficult to obtain topical formulations with the sough effect on sensorial perception and appearance.

Minoxidil is a potassium channel opener, which applied topically is widely used for the treatment of hair loss. It is effective in helping promote hair growth in both males and females with androgenetic alopecia.
The mechanism by which minoxidil promotes hair growth is not fully understood. The poor solubility of minoxidil makes difficult to obtain topical formulations with good cosmeticity.

In a particular embodiment, optionally in combination with one or more features of the particular embodiments defined above, the composition of the invention further comprises finasteride, more particularly in an amount from 0.2 to 1.5, particularly of 0.5 wt. %. In another particular embodiment, optionally in combination with one or more features of the particular embodiments defined above, the composition of the invention further comprises minoxidil in an amount from 1 to 5, particularly of 3 wt. %.

The composition of the invention can be prepared by conventional methods known to those skilled in the art, such as the one described in the examples. An exemplary process may include the addition of diaminopyrimidine oxide, oleanolic acid, biotinoyl tripeptide-1, apigenin, biotin, adenosine or an adenosine derivative, and Gingko biloba into a mixture of water and ethanol with conventional mixing techniques until the solution is homogeneous.

Throughout the description and claims the word "comprise" and variations of the word, are not intended to exclude other technical features, additives, components, or steps. Furthermore, the word "comprise" encompasses the case of "consisting of". The following examples are provided by way of illustration, and they are not intended to be limiting of the present invention. Furthermore, the present invention covers all possible combinations of particular and preferred embodiments described herein.

### EXAMPLES

### Example 1

This example illustrates an anti-hair loss lotion composition in accordance with the present invention.

| **Component** | **wt. %** |
|---|---|
| Purified water | q.s. |
| Denatured alcohol | 60.0 |
| Diaminopyrimidine oxide | 0.5 |
| Gingko biloba | 0.02 |
| Oleanolic acid | 0.001 |
| Biotinoyl tripeptide-1 | 0.0001 |
| PEG-40 Hydrogenated Castor oil | 0.50 |
| Apigen in | 0.008 |
| Adenosine | 0.2 |
| Biotin | 0.03 |
| Parfum (fragrance) | 0.02 |
| TOTAL | 100.0000 |

| | |
|---|---|
| q.s.: quantity sufficient to complete 100 g of topical composition | |

### Manufacturing Method:

A lotion was prepared by the addition of diaminopyrimidine oxide, oleanolic acid, biotinoyl tripeptide-1, adenosine, and Gingko biloba into a mixture of water and ethanol with conventional mixing techniques until the solution is homogeneous. The rest of components are also added to the mixture until an homogeneous solution is obtained.

### Cosmeticity

A study enrolling 20 men was carried out. The composition of Example 1 was applied with a slight massage onto the scalp of each one of the subjects. The cosmetic acceptability of the lotion was very good. After its application, the composition distributed uniformly without providing any oily, sticky feeling. The liquid topical composition had a fluid, watery texture and provided a non-greasy, non-sticky, fresh wet feeling. Additionally, the composition was quickly absorbed by the skin after its application, leaving a hair with a dry, clean, loose and flowing appearance. Furthermore, it was also stable.

### Example 2

This example illustrates an anti-hair loss lotion composition in accordance with the present invention. The lotion was prepared as disclosed in Example 1.

| **Component** | **wt. %** |
|---|---|
| Purified water | q.s. |
| Denatured alcohol | 46 |
| Diaminopyrimidine oxide | 1,5 |
| Gingko biloba | 0.1 |
| Oleanolic acid | 0.0009 |
| Biotinoyl tripeptide-1 | 0.0006 |
| PEG-40 Hydrogenated Castor oil | 1.00 |
| Apigen in | 0.0015 |
| Adenosine | 0.75 |
| Biotin | 0.01 |
| Parfum (fragrance) | 0.02 |
| TOTAL | 100.0000 |

| | |
|---|---|
| q.s.: quantity sufficient to complete 100 g of topical composition | |

A similar cosmeticity as for lotion of Example 1 was observed.

### Example 3

This example illustrates an anti-hair loss lotion composition in accordance with the present invention. The lotion was prepared as disclosed in Example 1.

| **Component** | **wt. %** |
|---|---|
| Purified water | q.s. |
| Denatured alcohol | 55.0 |
| Diaminopyrimidine oxide | 0,4 |
| Gingko biloba | 0.01 |
| Oleanolic acid | 0.0001 |
| Biotinoyl tripeptide-1 | 0.0001 |
| PEG-40 Hydrogenated Castor oil | 0.50 |
| Apigen in | 0.005 |
| Adenosine | 0.1 |
| Biotin | 0.003 |
| Parfum (fragrance) | 0.02 |
| TOTAL | 100.0000 |

| | |
|---|---|
| q.s.: quantity sufficient to complete 100 g of topical composition | |

A similar cosmeticity as for lotions of Example 1 and 2 was observed.

### Example 4.- Evaluation of synergistic effect of Ginkgo biloba on a combination of diaminopyrimidine oxide, oleanolic acid, biotinoyl tripeptide-1, apigenin, biotin, and adenosine

A study was conducted to evaluate the synergistic effect of the addition of Ginkgo biloba a hair lotion containing: diaminopyrimidine oxide, oleanolic acid, biotinoyl tripeptide-1, apigenin, biotin, and adenosine, in reducing hair loss in men with androgenetic alopecia.

### Subjects and methods

In a three-arm randomized study (1:1:1), subjects with type II and III androgenetic alopecia (AGA) were recruited; and were randomized to one of the following 3 groups: lotion with the sixactive agents diaminopyrimidine oxide, adenosine, oleanolic acid, biotinoyl tripeptide-1, apigenin and biotin (group A; as lotion of Example 2 without ginkgo biloba); lotion with ginkgo biloba (group B; as lotion of Example 2 without the rest of active agents); or lotion with the abovementioned six active agents + Ginko biloba (group C; lotion of Example 2). The lotion was applied on the scalp once daily for 6 months of treatment. The primary endpoint was the number of hairs in anagen and telogen phase by the use of TrichoScan® through 3 and 6 months of treatment.

### Results

In this study, 90 subjects with type II and III androgenetic alopecia (AGA) were included, of which 72 completed the study (24 subjects in group A, 23 in group B, and 25 in group C). The study showed a significant increase in the number of hairs in anagen phase after 3 and 6 months in the treatment groups A and C versus baseline, and at the same time a decrease in the number of hairs in telogen phase. Conversely, subjects in the treatment group B showed only a slight increase in the number of hairs in anagen phase and maintenance of hair in telogen phase during the study. Furthermore, the anagen/telogen ratio (A/T) showed significant improvement in both treatment groups (A and C) versus baseline at 3 and 6 months. In particular, subjects of treatment group C showed a significant improvement compared to group A both at 3 and at 6 months (p <0.01).

| | basal A/T | A/T 3 months | A/T 6 months |
|---|---|---|---|
| Group A (6 actives) | 1.82±0.13 | 1.95±0.14 | 2.1±0.14 |
| Group B (Gingko Biloba) | 1.80±0.14 | 1.82±0.14 | 1.84±0.12 |
| Group C (6 actives + GB) | 1.84±0.14 | 2.20±0.13 | 2.50±0.17 |

Surprisingly, it was also found that after discontinuing the treatment, the anagen/telogen ratio was significantly reduced to a lesser extent in group C compared to group A, and a significant increase in the number of hairs in the telogen phase in group A in relation to group C was observed. This was seen both at 3 and at 6 months after discontinuing the treatment.

## Claims

1. A combination comprising diaminopyrimidine oxide, oleanolic acid, biotinoyl tripeptide-1, apigenin, biotin, adenosine or an adenosine derivative, and Gingko biloba.

2. A topical composition comprising a therapeutically effective amount of the combination of claim 1, together with appropriate topical cosmetically or pharmaceutically acceptable excipients or carriers.

3. The composition according to claim 2, wherein the weight ratio of the combination diaminopyrimidine oxide, oleanolic acid, biotinoyl tripeptide-1, apigenin, biotin, and adenosine or an adenosine derivative to Ginkgo biloba is from 60:1 to 15:1, particularly from 50:1 to 20:1.

4. The composition according to claim 2, which is a lotion wherein the carrier is a mixture of water and ethanol, the water being present in an amount from 25 wt. % to 70 wt. % of the total composition, and the ethanol being present in an amount from 75 wt. % to 30 wt. % of the total composition.

5. The composition according to any one of claims 2 to 4, wherein the amount of diaminopyrimidine oxide is from 0.2 wt. % to 2 wt. %, particularly from 0.4 wt. % to 1.5 wt. %, of the total composition.

6. The composition according to any one of claims 2 to 5, wherein the amount of oleanolic acid is from 0.00001 wt. % to 0.1 wt. %, particularly from 0.0001 wt. % to 0.01 wt. %, of the total composition.

7. The composition according to any one of claims 2 to 6, wherein the amount of biotinoyl tripeptide-1 is from 0.00001 wt. % to 0.1 wt. %particularly from 0.0001 wt. % to 0.01 wt. % of the total composition.

8. The composition according to any one of claims 2 to 7, wherein the amount of apigenin is from 0.0001 wt. % to 0.01 wt. %, particularly from 0.0005 wt. % to 0.01 wt. %, of the total composition.

9. The composition according to any one of claims 2 to 8, wherein the amount of biotin is from 0.001 wt. % to 0.1 wt. %, particularly from 0.02 wt. % to 0.05 wt. %.

10. The composition according to any one of claims 2 to 9, wherein the amount of adenosine or adenosine derivative is from 0.1 wt. % to 1 wt. %, particularly from 0.20 wt. % to 1 wt. %, the total composition.

11. The composition according to claim 10, which comprises an adenosine 5'-phosphate derivative or a pharmaceutically acceptable salt thereof.

12. The composition according to any one of claims 2 to 11, wherein the amount of Gingko biloba is from 0.001 wt. % to 0.1 wt. %, particularly from 0.01 wt. % to 0.08 wt. %.

13. The composition according to any one of claims 2 to 12, further comprising finasteride.

14. The composition according to any one of claims 2 to 13, further comprising minoxidil.

15. The composition according to any one of claims 2 to 14, for use in reducing or preventing the loss of hair or stimulating its growth.

## Patentansprüche

1. Eine Kombination umfassend Diaminopyrimidinoxid, Oleanolsäure, Biotinoyl Tripeptid-1, Apigenin, Biotin, Adenosin oder ein Adenosinderivat, und Ginkgo biloba.

2. Eine topische Zusammensetzung umfassend eine therapeutisch wirksame Menge der Kombination des Anspruchs 1, zusammen mit geeigneten topischen kosmetisch oder pharmazeutisch akzeptablen Hilfsstoffen oder Trägersubstanzen.

3. Die Zusammensetzung nach Anspruch 2, wobei das Gewichtsverhältnis der Kombination aus Diaminopyrimidinoxid, Oleanolsäure, Biotinoyl Tripeptid-1, Apigenin, Biotin und Adenosin oder einem Adenosinderivat zu Ginkgo biloba von 60:1 bis 15:1, insbesondere von 50:1 bis 20:1 reicht.

4. Die Zusammensetzung nach Anspruch 2, welche eine Lotion ist, wobei die Trägersubstanz eine Mischung aus Wasser und Ethanol ist, wobei das Wasser in einer Menge von 25 Gew.-% bis 70 Gew.-% der gesamten Zusammensetzung vorhanden ist und das Ethanol in einer Menge von 75 Gew.-% bis 30 Gew.-% des gesamten Zusammensetzung vorhanden ist.

5. Die Zusammensetzung nach einem der Ansprüche 2 bis 4, wobei die Menge des Diaminopyrimidinoxids von 0,2 Gew.-% bis 2 Gew.-%, insbesondere von 0,4 Gew.-% bis 1,5 Gew.-% der gesamten Zusammensetzung reicht.

6. Die Zusammensetzung nach einem der Ansprüche 2 bis 5, wobei die Menge der Oleanolsäure von 0,00001 Gew.-% bis 0,1 Gew.-%, insbesondere von 0,0001 Gew.-% bis 0,01 Gew.-% der gesamten Zusammensetzung reicht.

7. Die Zusammensetzung nach einem der Ansprüche 2 bis 6, wobei die Menge des Biotinoyl Tripeptids-1 von 0,00001 Gew.-% bis 0,1 Gew.-%, insbesondere von 0,0001 Gew.-% bis 0,01 Gew.-% der gesamten Zusammensetzung reicht.

8. Die Zusammensetzung nach einem der Ansprüche 2 bis 7, wobei die Menge des Apigenins von 0,0001 Gew.-% bis 0,01 Gew.-%, insbesondere von 0,0005 Gew.-% bis 0,01 Gew.-% der gesamten Zusammensetzung reicht.

9. Die Zusammensetzung nach einem der Ansprüche 2 bis 8, wobei die Menge des Biotins von 0,001 Gew.-% bis 0,1 Gew.-%, insbesondere von 0,02 Gew.-% bis 0,05 Gew.-% reicht.

10. Die Zusammensetzung nach einem der Ansprüche 2 bis 9, wobei die Menge des Adenosins oder des Adenosinderivats von 0,1 Gew.-% bis 1 Gew.-%, insbesondere von 0,20 Gew.-% bis 1 Gew.-% der gesamten Zusammensetzung reicht.

11. Die Zusammensetzung nach Anspruch 10, welche ein Adenosin-5'-phosphat-Derivat oder ein pharmazeutisch akzeptables Salz davon umfasst.

12. Die Zusammensetzung nach einem der Ansprüche 2 bis 11, wobei die Menge von Ginkgo biloba von 0,001 Gew.-% bis 0,1 Gew.-%, insbesondere von 0,01 Gew.-% bis 0,08 Gew.-% reicht.

13. Die Zusammensetzung nach einem der Ansprüche 2 bis 12, weiterhin umfassend Finasterid.

14. Die Zusammensetzung nach einem der Ansprüche 2 bis 13, weiterhin umfassend Minoxidil.

15. Die Zusammensetzung nach einem der Ansprüche 2 bis 14, zur Verwendung bei der Reduktion oder der Prävention von Haarausfall oder bei der Stimulation des Haarwachstums.

## Revendications

1. Une combinaison comprenant de l'oxyde de diaminopyrimidine, de l'acide oléanolique, du biotinoyl tripeptide-1, de l'apigénine, de la biotine, de l'adénosine ou un dérivé d'adénosine et du Ginkgo biloba.

2. Une composition topique comprenant une quantité thérapeutiquement efficace de la combinaison de la revendication 1, avec des excipients ou des véhicules topiques cosmétiquement ou pharmaceutiquement acceptables appropriés.

3. La composition selon la revendication 2, dans laquelle le rapport en poids de la combinaison d'oxyde de diaminopyrimidine, acide oléanolique, biotinoyl tripeptide-1, apigénine, biotine, et adénosine ou un dérivé d'adénosine à Ginkgo biloba est de 60 : 1 à 15 : 1, en particulier de 50 : 1 à 20 : 1.

4. La composition selon la revendication 2, qui est une lotion dans laquelle le véhicule est un mélange d'eau et éthanol, l'eau étant présente dans une quantité de 25 % en poids à 70 % en poids de la composition totale et l'éthanol étant présent dans une quantité de 75 % en poids à 30 % en poids de la composition totale.

5. La composition selon l'une quelconque des revendications 2 à 4, dans laquelle la quantité d'oxyde de diaminopyrimidine est de 0,2 % en poids à 2 % en poids, en particulier de 0,4 % en poids à 1,5 % en poids de la composition totale.

6. La composition selon l'une quelconque des revendications 2 à 5, dans laquelle la quantité d'acide oléanolique est de 0,00001 % en poids à 0,1 % en poids, en particulier de 0,0001 % en poids à 0,01 % en poids de la composition totale.

7. La composition selon l'une quelconque des revendications 2 à 6, dans laquelle la quantité de biotinoyl tripeptide-1 est de 0,00001 % en poids à 0,1 % en poids, en particulier de 0,0001 % en poids à 0,01 % en poids de la composition totale.

8. La composition selon l'une quelconque des revendications 2 à 7, dans laquelle la quantité d'apigénine est de 0,0001 % en poids à 0,01 % en poids, en particulier de 0,0005 % en poids à 0,01 % en poids de la composition totale.

9. La composition selon l'une quelconque des revendications 2 à 8, dans laquelle la quantité de biotine est de 0,001 % en poids à 0,1 % en poids, en particulier de 0,02 % en poids à 0,05 % en poids.

10. La composition selon l'une quelconque des revendications 2 à 9, dans laquelle la quantité d'adénosine ou dérivé d'adénosine est de 0,1 % en poids à 1 % en poids, en particulier de 0,20 % en poids à 1 % en poids de la composition totale.

11. La composition selon la revendication 10, qui comprend un dérivé d'adénosine 5'-phosphate ou un sel pharmaceutiquement acceptable de celui-ci.

12. La composition selon l'une quelconque des revendications 2 à 11, dans laquelle la quantité de Ginkgo biloba est de 0,001 % en poids à 0,1 % en poids, en particulier de 0,01 % en poids à 0,08 % en poids.

13. La composition selon l'une quelconque des revendications 2 à 12, comprenant en outre du finastéride.

14. La composition selon l'une quelconque des revendications 2 à 13, comprenant en outre du minoxidil.

15. La composition selon l'une quelconque des revendications 2 à 14, pour l'utilisation dans la réduction ou la prévention de la perte de cheveux ou pour la stimulation de leur croissance.
